# EUROPEAN PATENT APPLICATION

(11) **EP 3 750 405 A1**
(43) Date of publication of application: **16.12.2020**
(21) Application number: 18908159.9
(22) Date of filing: 11.10.2018
(51) Int. Cl.: A23D 9/00, A23L 33/115, A23D 9/02, C11C 3/00, C11C 3/08, C12P 7/64

(54) **PRODUCTION METHOD FOR OIL/FAT COMPOSITION RICH IN PALMITIC ACID AT POSITION 2**

(30) Priority: 02.03.2018 JP 2018037071
(71) Applicant: Fuji Oil Holdings Inc., Izumisano-shi, Osaka 598-8540 (JP)
(72) Inventor: WATANABE, Shimpei, Tsukubamirai-shi, Ibaraki 200-2436 (JP)
(74) Representative: Boult Wade Tennant LLP
(86) International application number: PCT/JP2018/037928
(87) International publication number: WO 2019/167331

(57) **Abstract**

Oils and fats having a high 2-position palmitic acid content, which are used as a raw material for an oil/fat composition containing an XPX triglyceride having palmitic acid linked to position 2 thereof and X linked to positions 1 and 3 thereof, crystalize at low reaction temperature, likely leading to troubles such as clogging of a reaction vessel (X: an unsaturated fatty acid or a saturated fatty acid having not more than 10 carbon atoms). However, according to the present invention, when such oils and fats are mixed with a particular raw material fatty acid or a lower alcohol ester thereof, the cloud point of the raw material mixture can be set to 39.5°C or lower, and therefore, crystal deposition does not occur even at a low reaction temperature.

## Description

### Technical Field

The present invention relates to a method for producing a fat composition containing XPX triglyceride in which palmitic acid is at the 2-position and X is at the 1,3-positions. Here, X is an unsaturated fatty acid or saturated fatty acid having 10 or less carbon atoms, and XPX is a triglyceride in which palmitic acid is at the 2-position and X is at the 1,3-positions. In particular, the present invention relates to a method for producing a fat composition containing 1,3-dioleoyl-2-palmitoyl triglyceride (OPO).

### Background Art

Triacylglycerol is a major component of lipid which composes food, and is an important nutrient for a growth of living organism and an activity of living organism. In particular, 1,3-dioleoyl-2-palmitoyl triglyceride (OPO) is a major fat component of lipid contained in human milk.

A conventional food prepared for newborn baby, especially infant formula, contains a plurality of vegetable fats in order to mimic the fatty acid composition of fat in human milk. However, a binding sequence of fatty acids to glycerol backbone in a fat for infant formula prepared in this way differs greatly from the fat in human milk when attention is paid to the triacylglycerol structure. And, it has been pointed out that this difference may cause a nutritional difference for newborn having undeveloped digestive and absorptive organs.

A triacylglycerol structure of the fat contained in human milk is very characteristic. It is known that most of palmitic acid in constituent fatty acids of the fat exists in the form bound at the 2-position, and that unsaturated fatty acids, such as oleic acid, are bound at the 1,3-positions. However, in the case of an infant formula prepared by mixing vegetable fat, it is known that most of palmitic acid in constituent fatty acids of the fat is bound to the 1,3-positions and unsaturated fatty acid is bound to the 2-position. In addition, it has been widely studied that the above-mentioned structural difference brings important nutritional results (Non-Patent Documents 1 and 2). It has long been desired and studied to modify a fat for infant formula to a fat in which many of palmitic acid in constituent fatty acids of the fat is bound at the 2-position.

Patent Document 1 describes a method for producing a triglyceride having a high content of palmitic acid at the 2-position including subjecting a fatty triglyceride to random interesterification using a chemical catalyst, and then subjecting the fatty triglyceride to enzymatically interesterification with desired fatty acid to introduce the fatty acid into 1,3-positions using 1,3-position specific lipase.

Patent Document 2 describes a method for producing an OPO triglyceride including subjecting palm stearin having 18 to 40 of iodine value and containing tripalmitoyl glyceride to random interesterification, and then subjecting to an interesterification with using a 1,3-position specific lipase to introduce oleic acid into the 1,3-positions.

### Prior Art Documents

### Patent Documents

Patent Document 1: JP H06-70786 A
Patent Document 2: WO 2008/104381A

### Non-Patent Documents

Non-Patent Document 1: J. Nutr. 99, 293-298, (1969)
Non-Patent Document 2: J. Nutr. 95, 583-590, (1968)

### Summary of Invention

### Problems to Be Solved by Invention

In the process of studying an efficient method for producing a fat composition containing XPX triglyceride having X at the 1,3-positions and palmitic acid at the 2-position by a 1,3-position specific enzymatic interesterification, the present inventor has found that it is difficult to efficiently produce the fat composition when a random interesterified oil of palm stearin having 18 to 40 of iodine value as described in Patent Document 2 is used as a raw material fat because the content of palmitic acid at the 2-position is insufficient. In addition, the present inventor has found that it is difficult to efficiently produce the fat composition from the raw material fat disclosed in Patent Document 1 because the content of palmitic acid at the 2-position is insufficient similar to Patent Document 2. More specifically, Patent Document 1 does not particularly limit the content of palmitic acid at the 2-position in the chemically interesterified triglyceride used as the raw material fat, but the content of palmitic acid at the 2-position in the raw material fat in the Examples of Patent Document 1 is 59.3% by weight at the highest. As described above, it is necessary to use a fat having a high content of palmitic acid at the 2-position as a raw material in order to efficiently produce the fat composition. However, such fat is easy to be crystallized and may result in troubles such as clogging of reactor because an enzymatic interesterification reaction often requires a relatively low temperature condition.

When a temperature of interesterification reaction is set to be relatively high in order to avoid crystallization, there is a concern that an activity of the enzyme catalyst may decrease during repeated use and that a cost of the enzyme catalyst may increase.

An object of the present invention is to provide an efficient production method, which does not cause a problem such as clogging of reactor, by preventing crystallization of 1,3-specific enzymatic interesterification reaction solution in the raw material mixture and during the reaction when a temperature of the interesterification reaction is lowered.

### Means for Solving Problems

As a result of intensive study for solving the above problems, the present inventor has found that a fat composition containing XPX triglyceride is efficiently produced by mixing raw material fat having specific range of a content of palmitic acid at the 2-position of triglyceride of the fat and a specific raw material fatty acid or its lower alcohol ester (hereinafter referred to as "raw material fatty acid or ester"). By doing so, cloud point of the raw material mixture may be 39.5°C or lower, and fat crystallization does not occur even if a temperature of interesterification reaction is low. The present invention has been completed by these findings.

That is, the present invention relates to:
(1) A method for producing a fat composition containing XPX triglyceride, which includes preparing a raw material mixture by mixing a raw material fat and a raw material fatty acid or lower alcohol ester thereof, and subjecting the raw material mixture to a 1,3-position specific enzymatic interesterification, where a content of palmitic acid at 2-position of the raw material fat is 60 to 90% by weight, a constituent fatty acid of the raw material fatty acid or lower alcohol ester thereof is X, a cloud point of the raw material mixture and a reaction liquid from the 1,3-position specific enzymatic interesterification is 39.5°C or lower, and where X is an unsaturated fatty acid or saturated fatty acid having 10 or less carbon atoms and XPX is a triglyceride having palmitic acid at 2-position of the triglyceride and X at 1,3-positions of the triglyceride;
(2) The method for producing the fat composition of (1), where the raw material fat is a fat having 6 or more and less than 18 of iodine value obtained by subjecting a fat containing at least one kind of palm stearin to random interesterification;
(3) The method for producing the fat composition of (1), where the constituent fatty acid of the raw material fatty acid or lower alcohol ester thereof is unsaturated fatty acid;
(4) The method for producing the fat composition of (3), where the unsaturated fatty acid is oleic acid;
(5) The method for producing the fat composition of (1), where a mixing ratio of the raw material mixture (raw material fat/raw material fatty acid or lower alcohol ester thereof) is 5/95 to 40/60;
(6) The method for producing the fat composition of (1), where an acid value of the raw material fatty acid or lower alcohol ester thereof is 70 or less;
(7) The method for producing the fat of (1), where an acid value of the raw material fat is 2 or less.

### Effect of Invention

In the present invention, a mixing ratio of the raw material fatty acid or ester is necessary to be relatively high in order to make a cloud point of the raw material mixture 39.5°C or lower. As a result, the present invention may provide an effect that a fat composition having high purity of XPX triglyceride is easy to obtain.

### Mode for Carrying Out Invention

Hereinafter, the present invention will be described more specifically.

### (XPX triglyceride)

The present invention relates to a method for producing a fat composition containing XPX triglyceride having palmitic acid at the 2-position and X at the 1,3-positions. The fatty acids X at the 1,3-positions of XPX may be same or different. For example, XPX triglyceride includes triglyceride having palmitic acid at the 2-position and oleic acid at the 1,3-positions (OPO), and triglyceride having palmitic acid at the 2-position, oleic acid at one of the 1,3-positions, and DHA at the other position.

### (Content of palmitic acid at 2-position of raw material fat)

A raw material fat used in the present invention is necessary to meet that a content of palmitic acid at 2-position of triglyceride is 60 to 90% by weight. It is preferably 70% by weight or more, more preferably 76% by weight or more, and further preferably 78% by weight or more. And, it is preferably 88% by weight or less, more preferably 85% by weight or less, and further preferably 83% by weight or less.

When the content of palmitic acid at the 2-position is within this range, a fat composition containing XPX triglyceride may be efficiently produced, and a cloud point of the raw material mixture does not become too high.

### (Raw material fat)

A raw material fat used in the present invention is not particularly limited as long as a content of palmitic acid at the 2-position is in the above range. Examples of the raw material fat include vegetable fat such as palm oil, soybean oil, rapeseed oil, corn oil, cottonseed oil, peanut oil, sunflower oil, rice oil, safflower oil, high-oleic safflower oil, olive oil, sesame oil, coconut oil, and palm kernel oil; animal fat such as beef tallow, and lard; and processed fat thereof such as interesterified fat, fractionated fat, and hydrogenated fat; alone or in combination thereof. A fat obtained by subjecting a fat containing at least one kind of palm stearin to random interesterification is suitable for the raw material fat. An iodine value of the raw material fat is preferably 6 or more and less than 18. It is preferably 8 or more, more preferably 10 or more, further preferably 12 or more. Further, it is preferably 17 or less, more preferably 16 or less, further preferably 15 or less. A random interesterified fat from fat formulation, including two or more kinds of palm stearin having different qualities, palm oil, palm fractionated oil such as palm mid fraction, and/or fat other than palm related fat, may be used as the raw material fat.

Examples of the random interesterification include a method using a chemical catalyst such as sodium methylate and a method using a lipase having no position specificity as a catalyst. When a cloud point of fat is relatively high, a method using a chemical catalyst is preferable.

In addition, hydrogenation may be carried out before or after the interesterification. In this case, a fat composition rich in stearic acid content as well as a content of palmitic acid at the 2-position may be obtained.

### (Acid value of raw material fat)

An acid value of the raw material fat used in the present invention is preferably 2 or less. It is more preferably 1 or less, further preferably 0.5 or less, and most preferably 0.3 or less.

When the acid value is 2 or less, a cloud point of the raw material mixture becomes low, and it is preferable.

### (Fatty acid of raw material fatty acid or ester)

A constituent fatty acid of the raw material fatty acid or ester is necessary to be a saturated fatty acid or unsaturated fatty acid having 10 or less carbon atoms. In particular, unsaturated fatty acid is preferable. Examples of the unsaturated fatty acid include oleic acid, linoleic acid, linolenic acid, DHA, and EPA. In particular, oleic acid is more preferable because a fat composition containing OPO triglyceride, which is known to be contained in human milk at high content, is obtained and it has a high utility value as a raw material fat for a product such as infant formula.

The fatty acid lower alcohol ester of the present invention is not particularly limited as long as it is an ester of a fatty acid and an alcohol having 1 to 6 carbon atoms. The alcohol having 1 to 3 carbon atoms is preferable. Ethanol is more preferable.

### (Purity and acid value of specific fatty acid of raw material fatty acid or ester)

A constituent fatty acid of the raw material fatty acid or ester used in the present invention may be a mixture of a plurality of fatty acids, or may contain a single fatty acid in a purity of 50% by weight or more. The purity is preferably 60% by weight or more, more preferably 70% by weight or more, further preferably 80% by weight or more.

When a constituent fatty acid of the raw material fatty acid or ester is oleic acid, a content of oleic acid is not particularly limited, and preferably 70% by weight or more, more preferably 75% by weight or more, further preferably 80% by weight or more. A content of the palmitic acid in this case is not particularly limited, and preferably 10% by weight or less, more preferably 8% by weight or less, and further preferably 5% by weight or less. In addition, a raw material of oleic acid or oleic acid lower alcohol ester is not particularly limited as long as it is a vegetable fat that meets the above composition, preferably a fat having high oleic acid content such as high oleic sunflower oil and high oleic soybean oil.

The raw material fatty acid or ester used in the present invention may be a free fatty acid single product or a lower alcohol ester single product, and may be a mixture thereof. In the case of the mixture, acid value is preferably 70 or less, preferably 50 or less, more preferably 30 or less, further preferably 20 or less, and most preferably 10 or less.

When the acid value is 70 or less, that is, a proportion of the lower alcohol ester is high, a cloud point of the raw material mixture tends to be low, and it is preferable.

### (Cloud point)

A cloud point of the raw material mixture and reaction liquid of the 1,3-position specific enzymatic interesterification during and after the reaction in the present invention is necessary to be 39.5°C or lower. It is preferably 39°C or lower, more preferably 38°C or lower, further preferably 35°C or lower, and most preferably 30°C or lower.

### (Mixing ratio of raw material mixture)

A mixing ratio of the raw material mixture in the present invention (raw material fat/raw material fatty acid or ester) is preferably 5/95 to 40/60, by weight. It is more preferably 10/90 or more, further preferably 15/85 or more. In addition, it is more preferably 25/75 or less, further preferably 30/70 or less, and most preferably 35/65 or less.

When the mixing ratio of the raw material mixture is less than 5/95, production amount of the fat composition per raw material mixture tends to be small, and production efficiency tends to be poor. When it exceeds 40/60, the cloud point tends to be too high.

### (Lipase for 1,3-position specific enzymatic interesterification)

A lipase produced by a microorganism of genus *Rhizopus,* genus *Aspergillus,* or genus *Mucor* may be used for the 1,3-position specific enzymatic interesterification reaction. In addition, another lipase may be used as long as it has at least the same property as them. These lipases are commercially available, and lipase, such as Amano A (manufactured by Amano Pharmaceutical Co., Ltd.), and lipozyme (manufactured by NOVOZYMES), may be used. A use form of the above lipase is not particularly limited, and it is preferable to use with immobilizing it on a carrier by a known method from the viewpoint of efficiency. In addition, this reaction may be carried out by a batch method using a stirring tank or a continuous method using a packed reactor. It is particularly preferable to carry out by the continuous method using a packed reactor because a risk of clogging due to crystallization of the raw material mixture may be solved by the present invention.

### (Temperature and time of 1,3-position specific enzymatic interesterification reaction)

A temperature of the enzymatic reaction is preferably 30 to 90°C, more preferably 35 to 75°C, further preferably 40 to 55°C, from the viewpoint of maintaining a sufficient enzymatic reaction rate and maintaining the enzymatic activity for a long time, and from the viewpoint of avoiding the precipitation of fat crystal during the reaction, and the viewpoint of suppressing the production of isomer triglyceride as much as possible. In addition, the temperature is preferably at least 5°C higher than the cloud point of the raw material mixture from the viewpoint of avoiding precipitation of fat crystal during the reaction.

A time of the enzymatic reaction is not particularly limited as long as a sufficient interesterification rate may be achieved. And, it is preferably 2 hours to 4 days.

### (Other step)

A method for separating and removing the fatty acid or ester from the reaction liquid from the 1,3-position specific enzymatic interesterification reaction is not particularly limited, and distillation may be used. In addition, a purity of the fat composition containing XPX triglyceride obtained as the triglyceride fraction may be increased by fractionation.

### (Method for measuring cloud point)

In the present invention, a cloud point is measured by the following method according to the JOCS standard methods for analysis of fats and oils, 2.2.7-1996.
1. Sample is measured into a small test tube at 4.0 g and completely molten in a warm bath (at 60°C or higher for 15 minutes).
2. The small test tube of the above 1 is set to large test tube and move it to the measuring warm bath (set at 60°C).
3. Cooling is started when the fat reaches 60°C. Cooling rate is 0.25-0.3°C/min
4. Crystallization is visually confirmed, and the temperature is taken as the cloud point.

In the present invention, 39.5° C or lower of cloud point is regarded as acceptable, and a cloud point of higher than that is not acceptable.

### Examples

Examples will be described in the following. However, the spirit of the present invention is not limited by the examples. In the examples, any of part, ratio, and % means a weight basis.

### (Example 1)

Palm stearin (iodine value: 14.2) was subjected to random interesterification at 80°C for 30 minutes with adding sodium methylate at a content of 0.15% by weight with respect to the oil content, followed by washing with water according to a conventional method to obtain palm stearin random interesterrified oil. A content of palmitic acid at the 2-position of this oil was 80% by weight. An acid value of this oil was 0.24. A raw material mixture was obtained by mixing 20 parts by weight of this oil as a raw material fat and 80 parts by weight of ethyl oleate (content of oleic acid in the constituent fatty acids: 84% by weight, acid value: 8.8) as a raw material fatty acid or ester. A cloud point of the raw material mixture was 26.0°C, and it was acceptable. An interesetification reaction was carried out by passing the raw material mixture though a fixed bed reactor filled with 1.3-position specific lipase while maintaining the raw material mixture at 45°C. After the reaction, the obtained reaction solution was separated into a triglyceride fraction and a fatty acid fraction by distillation. A fatty acid composition of the obtained triglyceride fraction was that the content of palmitic acid in the total fatty acids was 36% by weight, and that the content of palmitic acid in the fatty acid at the 2-position was 76% by weight, and the composition was suitable as a fat for infant formula.

### (Example 2)

As a raw material fatty acid or ester, mixtures having specific acid value, sample A having 198.0 of acid value (single product of oleic acid), sample B having 50.0 of acid value, sample C having 30.0 of acid value, sample D having 20.0 of acid value, and sample E having 8.8 of acid value, were obtained by mixing ethyl oleate (purity: 80% by weight, acid value: 8.8) and oleic acid (purity: 80% by weight, acid value: 198.0). These raw material fatty acids or esters, samples A to E, were mixed with the random interesterified oil of the raw material fat obtained in Example 1, to adjust the ratio of raw material fat/raw material fatty acid or ester of the raw material mixture to 15/85, 20/80, 25/75, 30/70, 40/60 and 41.5/58.5, respectively. And, cloud point of the raw material mixture was measured. The results are shown in Table 1.

**Table 1 Cloud point (°C) of raw material mixture (iodine value of raw material fat: 14.2)**

| | | Raw material mixing ratio (weight ratio) | | | | | |
|---|---|---|---|---|---|---|---|
| Sample | Acid value | 15/85 | 20/80 | 25/75 | 30/70 | 40/60 | 41.5/ 58.5 |
| A | 198.0 | 28.5 | 32.6 | 33.0 | 37.0 | 39.2 | 40.0 |
| B | 50.0 | 23.5 | 27.8 | 31.2 | 31.5 | 38.2 | |
| C | 30.0 | 23.5 | 27.5 | 28.8 | 32.0 | 39.0 | |
| D | 20.0 | 23.5 | 27.0 | 28.9 | 31.0 | 35.5 | |
| E | 8.8 | 23.5 | 26.0 | 26.5 | 30.0 | 34.5 | |

The cloud point tended to increase as the mixing ratio of the raw material increases, and the cloud point tended to lower as the acid value of the raw material fatty acid or ester lower, that is, as the purity of the ethyl ester increases. In addition, a mixture of random interesterified oil of palm stearin (iodine value: 14.2) and sample A having 198.0 of acid value (single product of oleic acid) at 41.5/58.5 had a cloud point of 40.0°C and was not acceptable. However, the cloud points of all the other mixtures were 39.5°C or lower, and were acceptable.

### (Example 3)

A random interesterified oil of palm stearin was obtained in the same manner as in Example 1 except that palm stearin having 17.5 of iodine value was used. And it was used as a raw material fat.

Sample A (single product of oleic acid) having 198.0 of acid value was used as the raw material fatty acid or ester, and a raw material mixture was prepared so that the raw material fat/raw material fatty acid or ester was 40/60. The cloud point thereof was measured, and it was 36.0°C, and it was acceptable.

### (Example 4)

A random interesterified oil of palm stearin was obtained in the same manner as in Example 1 except that palm stearin having 6.0 of iodine value was used. And it was used as a raw material fat.

Sample A (single product of oleic acid) having 198.0 of acid value, sample B having 50.0 of acid value, and sample E having 8.8 of acid value were obtained as the raw material fatty acid or ester in the same manner as Example 2. Raw material mixtures were prepared by using the raw material fat and samples A, B, and E so that the raw material fat/raw material fatty acid or ester were 25/75, 30/70, and 40/60. The cloud point thereof was measured. The results are shown in Table 2.

**Table 2 Cloud point (°C) of raw material mixture (iodine value of raw material fat: 6.0)**

| | | Raw material mixing ratio (weight ratio) | | |
|---|---|---|---|---|
| Sample | Acid value | 25/75 | 30/70 | 40/60 |
| A | 198.0 | 35.1 | 39.1 | 40.9 |
| B | 50.0 | 32.0 | 37.5 | 39.0 |
| E | 8.8 | 31.4 | 36.0 | 39.0 |

The cloud point tended to increase as the mixing ratio of the raw material increases, and the cloud point tended to lower as the acid value of the raw material fatty acid or ester lower, that is, as the purity of the ethyl ester increases. A mixture of random interesterified oil of palm stearin (iodine value 6.0) and sample A (single product of oleic acid) having 198.0 of acid value had a cloud point of 40.9°C when the mixing ration of the raw material was 40/60. And, it was not acceptable. However, in the case of the mixture with the sample B having 50.0 of acid value, the result was 39.0°C. And, it was acceptable. Moreover, when the mixing ratio of the raw material was 25/70, and 30/70, all were acceptable.

### Industrial Applicability

The present invention enables to produce high-quality OPO fat with low cost because it enables to provide a smooth reaction process without crystallyzation even if a raw material fat having a high content of palmitic acid at the 2-position and having high cloud point is used in the step of 1,3-positin specific enzymatic interseterification in the production of OPO fat.

## Claims

1. A method for producing a fat composition containing XPX triglyceride, which comprises preparing a raw material mixture by mixing a raw material fat and a raw material fatty acid or lower alcohol ester thereof, and subjecting the raw material mixture to a 1,3-position specific enzymatic interesterification, wherein a content of palmitic acid at 2-position of the raw material fat is 60 to 90% by weight, a constituent fatty acid of the raw material fatty acid or lower alcohol ester thereof is X, a cloud point of the raw material mixture and a reaction liquid from the 1,3-position specific enzymatic interesterification is 39.5°C or lower, and wherein X is an unsaturated fatty acid or saturated fatty acid having 10 or less carbon atoms and XPX is a triglyceride having palmitic acid at 2-position of the triglyceride and X at 1,3-positions of the triglyceride.

2. The method for producing the fat composition according to claim 1, wherein the raw material fat is a fat having 6 or more and less than 18 of iodine value obtained by subjecting a fat containing at least one kind of palm stearin to random interesterification.

3. The method for producing the fat composition according to claim 1, wherein the constituent fatty acid of the raw material fatty acid or lower alcohol ester thereof is unsaturated fatty acid.

4. The method for producing the fat composition according to claim 3, wherein the unsaturated fatty acid is oleic acid.

5. The method for producing the fat composition according to claim 1, wherein a mixing ratio of the raw material mixture (raw material fat/raw material fatty acid or lower alcohol ester thereof) is 5/95 to 40/60.

6. The method for producing the fat composition according to claim 1, wherein an acid value of the raw material fatty acid or lower alcohol ester thereof is 70 or less.

7. The method for producing the fat according to claim 1, wherein an acid value of the raw material fat is 2 or less.
